(19) Europäisches Patentamt
European Patent Office
Office européen des brevets

(11) EP 4 509 124 A1

(12) **EUROPEAN PATENT APPLICATION**
published in accordance with Art. 153(4) EPC

(43) Date of publication:
**19.02.2025 Bulletin 2025/08**

(21) Application number: **22875213.5**

(22) Date of filing: **20.10.2022**

(51) International Patent Classification (IPC):
***A61K 31/519*** *(2006.01)* ***A61K 47/44*** *(2017.01)*
***A61K 47/24*** *(2006.01)* ***A61K 47/10*** *(2017.01)*
***A61K 9/06*** *(2006.01)* ***A61P 19/08*** *(2006.01)*
***A61P 7/00*** *(2006.01)*

(52) Cooperative Patent Classification (CPC):
**A61K 31/519; A61K 9/0014; A61K 9/06; A61K 47/14; A61K 47/38; A61P 7/00; A61P 17/06; A61P 19/08;** Y02A 50/30

(86) International application number:
**PCT/CN2022/126399**

(87) International publication number:
**WO 2023/051833 (06.04.2023 Gazette 2023/14)**

(84) Designated Contracting States:
**AL AT BE BG CH CY CZ DE DK EE ES FI FR GB GR HR HU IE IS IT LI LT LU LV MC ME MK MT NL NO PL PT RO RS SE SI SK SM TR**

(30) Priority: **28.09.2021 CN 202111142379**

(71) Applicant: **Hangzhou Zhongmeihuadong Pharmaceutical Co., Ltd.**
**GongShu District**
**HangZhou, Zhejiang 310011 (CN)**

(72) Inventors:
- **YU, Jin**
  **Hangzhou, Zhejiang 310011 (CN)**
- **LUO, Yanhua**
  **Hangzhou, Zhejiang 310011 (CN)**
- **QI, Weirui**
  **Hangzhou, Zhejiang 310011 (CN)**
- **WU, Xiaoxiao**
  **Hangzhou, Zhejiang 310011 (CN)**
- **YAN, Tingting**
  **Hangzhou, Zhejiang 310011 (CN)**

(74) Representative: **Nederlandsch Octrooibureau**
**P.O. Box 29720**
**2502 LS The Hague (NL)**

# (54) RUXOLITINIB COMPOSITION AND PREPARATION METHOD THEREFOR

(57) Disclosed in the present invention is a Ruxolitinib composition, comprising: Ruxolitinib or a pharmaceutically acceptable salt, an emulsifier, a co-emulsifier, a solvent, and an aqueous phase thereof. The emulsifier comprises one or more of polyoxyethylene hydrogenated castor oil, polyoxyethylene castor oil and vitamin E succinic acid. The co-emulsifier comprises propylene glycol monocaprylate. The solvent comprises one or more of propylene glycol, diethylene glycol monoethyl ether, and ethanol. The Ruxolitinib composition of the present invention has an ideal particle size and PDI. Furthermore, the present invention provides Ruxolitinib gel. Compared with a Ruxolitinib cream, the film-permeable efficiency is improved, and the stability of administration to the external skin under the condition of pH 5-7 can be satisfied; compared with the Ruxolitinib cream, as well as commercially available calcipotriol and Benvitimod, the present invention achieves a better drug effect in the aspect of psoriasis treatment.

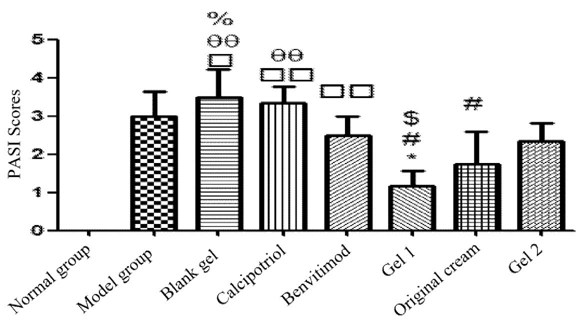


FIG.1

EP 4 509 124 A1

## Description

### Field of the invention

The present invention relates to the field of drug formulations, in particular to a composition of ruxolitinib or a pharmaceutically acceptable salt thereof, and a preparation method for the composition.

### Background of the invention

Ruxolitinib, with the chemical name of (R)-3-cyclopentyl-3-[4-(7H-pyrrolo [2,3-d]pyrimidin-4-yl)-1H-pyrazol-1-yl] propionitrile, is an inhibitor for tyrosine kinases JAK1 and JAK2, for used in adult patients with middle- or high-risk primary myelofibrosis (PMF) (also known as chronic idiopathic myelofibrosis), myelofibrosis secondary to polycythemia vera (PPV-MF), or myelofibrosis secondary to primary thrombocytosis (PET-MF) for treating splenomegaly or other symptoms associated with the above diseases.

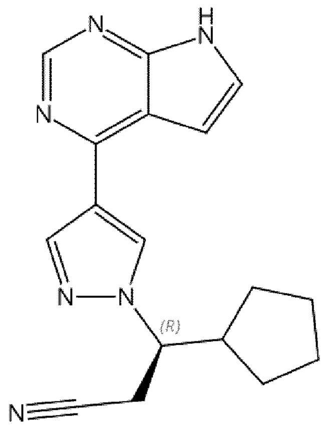

Structural formula of ruxolitinib

The Chinese invention patent application with publication No. CN103002875B discloses a cream formed by an oil-in-water emulsion for topical skin application. The cream has (R)-3-cyclopentyl-3-[4-(7H-pyrrolo[2,3-d]pyrimidin-4-yl)-1H-pyrazol-1-yl] propionitrile (i.e. ruxolitinib) or a pharmaceutically acceptable salt thereof as the therapeutic agent, an oil phase selected from one or more of vaseline, fatty alcohols, mineral oils, triglycerides, and dimethyl siloxane, an emulsifier component including one or more independently selected from glycerol fatty esters and sorbitan fatty acid esters, a solvent component including one or more independently selected from alkylene glycols and polyalkylene glycols, and a stabilizer component including one or more independently selected from polysaccharides. Topical formulations generally require a pH value between 4.5 and 7, while the sample of the formulation in that patent has a pH value of about 3.1-3.6, so that the formulation has certain skin irritation to the patients when applied, and is not suitable for skin sites with certain damage.

The US invention patent with publication No. US20200405627A1 discloses a method for treating vitiligo by using 1.5% w/w oil-in-water emulsion of ruxolitinib, which has an oil component selected from vaseline, fatty alcohols, mineral oils, triglycerides, and polydimethylsiloxane, an emulsifier component selected from glycerol fatty acid esters and sorbitan fatty acid esters, a solvent component selected from alkylene glycols and polyalkylene glycols, and a stabilizer component selected from polysaccharides such as xanthan gum.

*In vitro* release test is a currently recognized effective method for evaluating semi-solid formulations (such as a cream, an ointment, and a gel), and is an *in vitro* manifestation of the efficacy of the formulations *in vivo*. *In vitro* release is characterized by strong operability, good reproducibility and high sensitivity. Therefore, a suitable *in vitro* release test method will facilitate the development of transdermal absorption formulations. The Chinese invention patent with publication No. CN111337640A discloses a method for rapidly evaluating *in vitro* release of a topical formulation. At present, *in vitro* release and penetration of topical formulations is mainly evaluated in a Franz diffusion cell. *In vitro* release is conducted using an artificial membrane, while penetration is conducted using isolated rat abdominal skin or Bama miniature pig skin, or an artificial bionic membrane (Strat-M® Membrane) to investigate skin penetration rate of drugs.

**Summary of the Invention**

In one aspect, the present invention provides a ruxolitinib composition containing a therapeutic agent and an emulsifier.

In some embodiments according to the invention, the therapeutic agent is ruxolitinib and/or a pharmaceutically acceptable salt thereof, with a free base-based content of 0.5%-2.0% of the weight of the composition.

In some embodiments according to the invention, the emulsifier is one or more of polyoxyethylene alkyl aryl ether, polyoxyethylene monolaurate, polyoxyethylene monolaurate, polyoxyethylene lauryl ether, polyoxyethylene hydrogenated castor oil, polyoxyethylene castor oil, polyoxyethylene plant oil, polyoxyethylene monooleate, polyoxyethylene monolaurate, and vitamin E succinate, preferably one or more of polyoxyethylene hydrogenated castor oil, polyoxyethylene castor oil, polyoxyethylene monooleate, polyoxyethylene monolaurate, and vitamin E succinate, and further preferably one or more of polyoxyethylene hydrogenated castor oil, polyoxyethylene castor oil, and vitamin E succinate.

In some embodiments according to the invention, the emulsifier is one or more of polyoxyethylene hydrogenated castor oil, polyoxyethylene castor oil, and vitamin E succinate, wherein the polyoxyethylene hydrogenated castor oil is one or more selected from RH20, RH30, RH40, RH50, and RH60, preferably one of RH40 and RH60, and more preferably RH40; the polyoxyethylene castor oil is one or more selected from EL20, EL25, EL30, EL35, EL40, and EL60, preferably one or more of EL30, EL35, and EL40, and more preferably EL35; and the vitamin E succinate is one or more selected from VE-TPGS 400, VE-TPGS 1000, and VE-TPGS1500, preferably VE-TPGS 1000. The content of the emulsifier is 2%-30%, further preferably 3%-12% of the weight of the composition.

In some embodiments according to the invention, the ruxolitinib composition contains a therapeutic agent, an emulsifier, and a co-emulsifier.

In some embodiments according to the invention, the co-emulsifier contains propylene glycol monocaprylate (Capryol-90), with a content of 0-10%, preferably 0-7% of the weight of the composition.

In some embodiments according to the invention, the ruxolitinib composition contains a therapeutic agent, an emulsifier, a co-emulsifier, and a solvent.

In some embodiments according to the invention, the solvent is one or more of propylene glycol, diethylene glycol monoethyl ether (HP), and ethanol, with a content of 0-60%, preferably 0-50% of the weight of the composition.

In some embodiments according to the invention, the ruxolitinib composition contains a therapeutic agent, an emulsifier, a co-emulsifier, a solvent, and an aqueous phase.

In some embodiments according to the invention, the aqueous phase contains water and optionally an excipient. A pH regulator, an osmotic pressure regulator, a preservative, an antioxidant, an opacifying agent or the like can be added to water to further improve a formulation of the ruxolitinib composition in order to meet the clinical demands of different administration routes, and the content of the aqueous phase is such that it, together with the contents of the therapeutic agent, the emulsifier, the co-emulsifier and the solvent, reaches a sum of 100% of the weight of the composition.

In some embodiments of the present invention, the ruxolitinib composition contains:

a therapeutic agent: ruxolitinib, or a pharmaceutically acceptable salt thereof;
an emulsifier: one or more of polyoxyethylene hydrogenated castor oil, polyoxyethylene castor oil, and vitamin E succinate;
a co-emulsifier: propylene glycol monocaprylate;
a solvent: one or more of propylene glycol, diethylene glycol monoethyl ether, and ethanol; and
water q.s. to 100wt%.

In some embodiments of the present invention, the ruxolitinib composition contains:

a therapeutic agent: 0.5%-2.0% of ruxolitinib, or a pharmaceutically acceptable salt thereof (based on the free base);
an emulsifier: 2%-30%, further preferably 3%-12% of one or more of polyoxyethylene hydrogenated castor oil, polyoxyethylene castor oil, and vitamin E succinate;
a co-emulsifier: 0-10%, preferably 0-7% of propylene glycol monocaprylate;
a solvent: 0-60%, preferably 0-50% of one or more of propylene glycol, diethylene glycol monoethyl ether, and ethanol; and
water q.s. to100wt%,
based on the total weight of the composition.

**In** an embodiment according to the invention, a method for preparing the ruxolitinib composition is as follows:

1) heating the therapeutic agent, the emulsifier, and the co-emulsifier to 30-70°C, preferably 35-60°C, stirring, followed by adding the aqueous phase to obtain the ruxolitinib composition; or

2) mixing the therapeutic agent and the co-emulsifier, heating them to 30-70°C, preferably 35-60°C while stirring until dissolved, then adding the emulsifier, stirring, followed by adding the aqueous phase to obtain the ruxolitinib composition.

**In** an embodiment according to the invention, the method for preparing the ruxolitinib composition is as follows:

1) heating the therapeutic agent, the emulsifier, the solvent, and the co-emulsifier to 30-70°C, preferably 35-60°C, stirring, followed by adding the aqueous phase to obtain the ruxolitinib composition; or
2) heating the therapeutic agent, the co-emulsifier, and the solvent to 30-70°C, preferably 35-60°C while stirring until dissolved, then adding the emulsifier, stirring, followed by adding the aqueous phase to obtain the ruxolitinib composition.

In another aspect of the present invention, the present invention provides a use of the ruxolitinib composition as described above. The composition can be used for preparing a pharmaceutical formulation, including but not limited to a tablet, granules, micro pills, an ointment, a cream, and a gel.

In yet another aspect of the present invention, the present invention provides a use of the ruxolitinib composition as described above, particularly a ruxolitinib gel. In addition to the ruxolitinib composition as described above, the gel contains a gel material. The gel material is one or more selected from carbomer, methylcellulose, sodium carboxymethyl cellulose, chitosan, gellan gum, guar gum, sodium carboxymethyl cellulose, and hydroxypropyl cellulose, preferably carbomer, with a content of 0.10%-2.0% in percentage by weight, further preferably 0.25%-1.0%.

In some embodiments, the ruxolitinib gel contains:

a therapeutic agent: the ruxolitinib, or a pharmaceutically acceptable salt thereof;
an emulsifier: one or more of polyoxyethylene hydrogenated castor oil, polyoxyethylene castor oil, and vitamin E succinate;
a co-emulsifier: propylene glycol monocaprylate;
a solvent: one or more of propylene glycol, diethylene glycol monoethyl ether, and ethanol;
a gel material: one or more of carbomer, methylcellulose, sodium carboxymethyl cellulose, chitosan, gellan gum, guar gum, sodium carboxymethyl cellulose, and hydroxypropyl cellulose; and
water q.s. to 100wt%.

In some embodiments, the ruxolitinib gel contains:

a therapeutic agent: 0.5%-2.0% of ruxolitinib, or a pharmaceutically acceptable salt thereof (based on the free base);
an emulsifier: 2%-30%, preferably 3%-12% of one or more of polyoxyethylene hydrogenated castor oil, polyoxyethylene castor oil, and vitamin E succinate;
a co-emulsifier: 0-10%, preferably 0-7% of propylene glycol monocaprylate;
a solvent: 0-60%, preferably 0-50%, more preferably 2%-50% of one or more of propylene glycol, diethylene glycol monoethyl ether, and ethanol;
a gel material: 0.10%-2.0%, preferably 0.25%-1.0% of one or more of carbomer, methylcellulose, sodium carboxymethyl cellulose, chitosan, gellan gum, guar gum, sodium carboxymethyl cellulose, and hydroxypropyl cellulose; and
water q.s. to 100wt%;
based on the total weight of the gel.

In some embodiments, the ruxolitinib gel contains:

a therapeutic agent: 0.5%-2.0% of ruxolitinib, or a pharmaceutically acceptable salt thereof (based on the free base);
an emulsifier: 1%-10% of polyoxyethylene hydrogenated castor oil, 1%-20% of polyoxyethylene castor oil, and 0-20% of vitamin E succinate;
a co-emulsifier: 0-20% of propylene glycol monocaprylate;
a solvent: 1%-50% of propylene glycol, 1%-10% of diethylene glycol monoethyl ether, and 0-50% of ethanol;
a gel material: 0.10%-10.0% of carbomer; and
water q.s. to 100wt%;
based on the total weight of the gel.

In some embodiments, the ruxolitinib gel contains:

a therapeutic agent: 0.5%-2.0% of ruxolitinib, or a pharmaceutically acceptable salt thereof (based on the free base);
an emulsifier: 1.0%-5.0% of polyoxyethylene hydrogenated castor oil, 1%-10% of polyoxyethylene castor oil, and 0-10% of vitamin E succinate;
a co-emulsifier: 0-10% of propylene glycol monocaprylate;
a solvent: 1%-45% of propylene glycol, 1%-5.0% of diethylene glycol monoethyl ether, and 0-40% of ethanol;
a gel material: 0.10%-5.0% of carbomer; and
water q.s. to 100wt%;
based on the total weight of the gel.

In some embodiments, the ruxolitinib gel contains:

a therapeutic agent: 0.5%-2.0% of ruxolitinib, or a pharmaceutically acceptable salt thereof (based on the free base);
an emulsifier: 1%-2% of polyoxyethylene hydrogenated castor oil, 4%-10% of polyoxyethylene castor oil, and 0-5% of vitamin E succinate;
a co-emulsifier: 0-7% of propylene glycol monocaprylate;
a solvent: 5%-45% of propylene glycol, 2%-5% of diethylene glycol monoethyl ether, and 0-15% of ethanol;
a gel material: 0.25%-1.0% of carbomer; and
water q.s. to 100wt%;
based on the total weight of the gel.

According to an embodiment of the invention, the method for preparing the gel of the ruxolitinib composition includes the following steps: mixing the ruxolitinib composition except the aqueous phase and heating to 30-70°C, preferably 35-60°C, stirring to make the excipient completely melted and the therapeutic agent dissolved, then uniformly stirring with swollen carbomer, followed by adding the aqueous phase to obtain the ruxolitinib gel.

In yet another aspect, the present invention provides a use of the ruxolitinib composition and the ruxolitinib gel as described above for treating diseases which is one or more of dermatitis, psoriasis, vitiligo, hydradenitis, urticaria, and alopecia areata.

The present invention has the following beneficial effects: the ruxolitinib composition provided in the present invention has an average particle size ranging from 10 to 200 nm and a PDI value of 0.05-0.40, and further can reach an average particle size ranging from 10 to 100 nm and a PDI value of 0.05-0.20; and is characterized by good stability and being less prone to aggregation and flocculation, etc. Further, the gel prepared from a composition of the present invention has improved membrane penetration efficiency, and satisfactory stability when topically administered to the skin at a pH value of 5-7, compared to a ruxolitinib cream. Even further, the gel prepared from a composition of the present invention has better efficacy in the treatment of psoriasis, compared to a ruxolitinib cream and commercially available calcipotriol and Benvitimod.

The solutions of the present invention will be explained below in combination with Examples. Those skilled in the art will understand that the following examples are only for illustrating the present invention, and should not be taken as limiting the scope of the present invention. Where specific technologies or conditions are not noted in the Examples, the technologies or conditions described in the literatures in the art or the instructions of products are followed. All agents or instruments that are not noted with manufacturers are conventional products that are commercially available.

## Brief Description of the Drawings

FIG. 1 is a diagram showing PASI scores 7 days after administration in Example 6; and

FIG. 2 is a plot of PASI appearance scores in Example 6.

## Detailed Description of the Invention

All the reagents and raw materials used in the following Examples are commercially available.

"Diethylene glycol monoalkyl ether" should be understood as one or more diethylene glycols substituted by C1 to C6 alkyl ethers. Diethylene glycol monoalkyl ethers is one or two of diethylene glycol monoethyl ether (DGME) and diethylene glycol monomethyl ether (DGMM).

HP in the Examples of the present invention refers to diethylene glycol monoalkyl ethers, further to diethylene glycol monoethyl ether.

In the present invention, "PDI" is the abbreviation of polydispersity index, and its physical meaning is the

uniformity of particle size distribution. The smaller a value (distribution range) is, the more uniform the particle size dispersion is.

The average particle size and the PDI value of the ruxolitinib composition were detected by Malvern Nano S90.

**Example 1: Ruxolitinib composition and method for preparation thereof**

I. Prescription and constitution

The ruxolitinib composition of the present invention contains the following components:

a therapeutic agent: ruxolitinib (its content was calculated based on the free base),
an emulsifier: one or more of polyoxyethylene hydrogenated castor oil, polyoxyethylene castor oil, and vitamin E succinate,
a co-emulsifier: propylene glycol monocaprylate (Capryol-90),
a solvent: one or more of propylene glycol, diethylene glycol monoethyl ether (HP), and ethanol,
an aqueous phase: containing water and pharmaceutically acceptable excipients, wherein the excipients include, but not limited to, a pH regulator, an osmotic pressure regulator, a preservative, an antioxidant, and an opacifying agent.

II. Preparation of the ruxolitinib composition

Prescriptions 1-7 were prepared with Method 1: mixing the therapeutic agent, the emulsifier, the co-emulsifier and the solvent, heating them to 50°C and stirring to make the excipients completely melted and the therapeutic agent dissolved, followed by adding the aqueous phase to obtain the ruxolitinib composition.

Prescriptions 8-14 were prepared with Method 2: mixing the therapeutic agent, the co-emulsifier and the solvent, heating them to 50°C and stirring to make the excipients completely melted and the therapeutic agent dissolved, then adding the emulsifier, stirring, followed by adding the aqueous phase to obtain the ruxolitinib composition.

Table 1: Investigation on proportions of the components

| | Prescript ion 1 | Prescript ion 2 | Prescript ion 3 | Prescript ion 4 | Prescript ion 5 | Prescript ion 6 | Prescript ion 7 |
|---|---|---|---|---|---|---|---|
| Therapeut ic agent | 0.5% | 0.75% | 1.00% | 2.00% | 1.00% | 1.00% | 1.00% |
| Emulsifier | RH40: 10% | RH40: 9.25% | RH40: 9% | RH40: 8% | EL35: 4% | RH40: 1% EL35: 4% | RH40: 1% EL35: 4% |
| Co-emulsi fier | 6.00% | 5.00% | 5.00% | 5.00% | 0.00% | 0.00% | 0.00% |
| Solvent | HP: 5% Propylen e glycol: 30% | Propylen e glycol: 30% | Propylen e glycol: 30% | Propylen e glycol: 30% | HP: 2% | HP: 2% | HP: 2% Propylen e glycol: 15% |
| Aqueous phase | 54.25% | 55% | 55% | 55% | 93.00% | 92.00% | 77.00% |
| Average particle size | 61.12nm | 19.73nm | 20.04nm | 68.49nm | 40.25nm | 13.61nm | 14.28nm |
| PDI | 0.044 | 0.035 | 0.027 | 0.2 | 0.189 | 0.133 | 0.101 |

Table 2: Investigation on proportions of the components

| | Prescrip tion 8 | Prescrip tion 9 | Prescrip tion 10 | Prescripti on11 | Prescripti on12 | Prescrip tion 13 | Prescrip tion 14 |
|---|---|---|---|---|---|---|---|
| Therapeut ic agent | 1.00% | 1.00% | 1.00% | 1.00% | 1.00% | 1.00% | 2.00% |

(continued)

| | Prescrip tion 8 | Prescrip tion 9 | Prescrip tion 10 | Prescripti on11 | Prescripti on12 | Prescrip tion 13 | Prescrip tion 14 |
|---|---|---|---|---|---|---|---|
| Emulsifie r | RH40: 1% EL35: 10% | RH40: 1% EL35: 10% | RH40: 1% EL35: 4% | VE-TPGS 1000: 5% | RH40: 1% EL35: 4% | RH40: 1% EL35: 4% | RH40: 2% EL35: 10% |
| Co-emulsifier | 0.00% | 0.00% | 0.00% | 2.00% | 3.00% | 5.00% | 7.00% |
| Solvent | HP: 5% Propyle ne glycol: 15% | HP: 5% | HP: 2% Propyle ne glycol: 45% | HP: 5% Propylene glycol: 5% | HP:2% Propylene glycol: 15% | Propyle ne glycol: 30% | Propyle ne gly-col: 27.5% Ethanol: 14% |
| Aqueous phase | 68.00% | 83% | 67% | 82% | 74% | 59% | 45.00% |
| Average particle size | 13.67nm | 13.02nm | 14.14nm | 13.29nm | 40.55nm | 42.13nm | 50.01nm |
| PDI | 0.094 | 0.023 | 0.066 | 0.042 | 0.057 | 0.096 | 0.134 |

As shown in Table 1 and Table 2, when the content of the therapeutic agent was 0.5-2.0%, the emulsifier could be one or more of polyoxyethylene hydrogenated castor oil, polyoxyethylene castor oil, and vitamin E succinate, with a content of 3%-12% w/w; the content of the co-emulsifier was 0-7% w/w; the content of the solvent was 2%-50% w/w; and the sum of the contents of the aqueous phase, the therapeutic agent, the emulsifier, the co-emulsifier and the solvent was 100%. The resultant ruxolitinib composition had an average particle size of not more than 100 nm and a PDI value of not more than 0.2.

**Example 2: Investigation on Different Emulsifiers**

The preparation method of Example 2 was the same as that of Example 1, except the type and amount of the emulsifier used, particularly as shown in the following table:

Table 3. Investigation by Comparative Examples

| | | Compara tive example 1 | Compara tive example 2 | Compara tive example 3 | Compara tive example 4 | Compara tive example 5 | Compara tive example 6 |
|---|---|---|---|---|---|---|---|
| Thera peutic agent | Ruxolitinib free base | 1% | 1% | 1% | 1% | 1% | 1% |
| Emuls ifier | Kolliphor hs15 | 5% | / | / | / | | |
| | Glycerol fatty acid ester | / | 10% | / | / | / | / |
| | Poloxamer 407 | / | / | 10% | / | / | / |
| | Tween 80 | / | / | / | 10% | / | / |
| | Polyoxyethlen e cetyl ether | / | / | / | / | 10% | / |
| | Polyoxyethyle ne sorbitan monolaurate | / | / | / | / | / | 10% |
| Co-e mulsif ier | Capryol-90 | 7% | 7% | 7% | 7% | 7% | 7% |

(continued)

| | | Compara tive example 1 | Compara tive example 2 | Compara tive example 3 | Compara tive example 4 | Compara tive example 5 | Compara tive example 6 |
|---|---|---|---|---|---|---|---|
| Solvent | HP | 5% | 5% | 5% | 5% | 5% | 5% |
| | Propylene glycol | 15% | 15% | 15% | 15% | 15% | 15% |
| Aqueous phase | Purified water | 62% | 62% | 62% | 62% | 62% | 62% |
| Average particle size | | 605nm | 504nm | 526nm | 483nm | Milky white, layered, visually larger particle size | Milky white, layered, visually larger particle size |
| PDI value | | 0.534 | 0.534 | 0.601 | 0.516 | | |

As shown in Table 3, when the emulsifier was selected from Kolliphor hs15, glycerol fatty acid ester, poloxamer 407, Tween 80, polyoxyethlene cetyl ether, and polyoxyethylene sorbitan monolaurate, the ruxolitinib composition with a desirable particle size or PDI value could not be formed.

**Example 3: Investigation on stability of prescriptions of the ruxolitinib composition**

Prescription 2, Prescription 8, Prescription 9, and Prescription 12 of the ruxolitinib composition in Example 1 were selected. The stability of the ruxolitinib composition prescriptions of the present invention was investigated based on the change in average particle size and PDI value of the ruxolitinib composition prescriptions of the present invention after one month and two months at 25°C and 40°C. Detailed results are shown in Table 4.

Table 4: Stability of the ruxolitinib compositions

| Sample at 25°C | Day 0 | | | | 1 M at 25°C | | | 2 M at 25°C | | |
|---|---|---|---|---|---|---|---|---|---|---|
| Batch No. | pH | Average particle size (nm) | PDI | Content (mg/ml) | Average particle size (nm) | PDI | Content (mg/ml) | Average particle size (nm) | PDI | Content (mg/ml) |
| Prescription 2 | 6.087 | 19.73 | 0.035 | 10.88 | 21.59 | 0.026 | 10.79 | 21.28 | 0.020 | 10.79 |
| Prescription 8 | 6.632 | 13.33 | 0.041 | 10.92 | 13.5 | 0.041 | 11.03 | 13.43 | 0.030 | 10.79 |
| Prescription 9 | 6.539 | 13.02 | 0.023 | 10.95 | 12.82 | 0.031 | 10.86 | 12.85 | 0.021 | 10.91 |
| Prescription 12 | 6.411 | 14.13 | 0.058 | 10.99 | 14.85 | 0.043 | 10.80 | 18.83 | 0.147 | 10.86 |
| Sample at 40°C | Day 0 | | | | 1 M at 40°C | | | 2 M at 40°C | | |
| Batch No. | pH | Average particle size (nm) | PDI | Content (mg/ml) | Average particle size (nm) | PDI | Content (mg/ml) | Average particle size (nm) | PDI | Content (mg/ml) |
| Prescription 2 | 6.087 | 19.73 | 0.035 | 10.88 | 21.43 | 0.025 | 10.79 | 20.99 | 0.023 | 10.79 |

(continued)

| Sample at 40°C | Day 0 | | | | 1 M at 40°C | | | 2 M at 40°C | | |
|---|---|---|---|---|---|---|---|---|---|---|
| Batch No. | pH | Average particle size (nm) | PDI | Content (mg/ml) | Average particle size (nm) | PDI | Content (mg/ml) | Average particle size (nm) | PDI | Content (mg/ml) |
| Prescription 8 | 6.632 | 13.33 | 0.041 | 10.92 | 13.53 | 0.027 | 10.77 | 13.39 | 0.020 | 10.96 |
| Prescription 9 | 6.539 | 13.02 | 0.023 | 10.95 | 13.06 | 0.024 | 10.63 | 12.86 | 0.021 | 10.89 |
| Prescription 12 | 6.411 | 14.13 | 0.058 | 10.99 | 15.04 | 0.049 | 10.83 | 15.05 | 0.059 | 10.88 |

The above results show the ruxolitinib compositions provided in the present invention underwent no significant change in the average particle size or the PDI at room temperature or 40°C, and had good stability.

In addition, Prescription 12 of the ruxolitinib composition in Example 1 was selected for investigating pH stability of the ruxolitinib composition prescription of the present invention. Prescription 12 was adjusted to different pH values using triethanolamine, and subjected to experiments at room-temperature or under accelerated conditions to investigate the content of the ruxolitinib in the prescription. Detailed results are shown in Table 5.

Table 5: pH Stability of Ruxolitinib Composition Prescription

| Batch No. | pH on Day 0 | | Day 0 | 30 days at 40°C | 30 days at room temperature | 60 days at 40°C | 60 days at room temperature |
|---|---|---|---|---|---|---|---|
| | Before adjustment | After adjustment | | | | | |
| Prescription 12 | 6.615 | 3.251 | 9.53 mg/g | 9.51 mg/g | 9.39 mg/g | 9.60 mg/g | 9.66 mg/g |
| Prescription 12 | 6.701 | 4.855 | 9.62 mg/g | 9.55 mg/g | 9.59 mg/g | 9.91 mg/g | 9.52 mg/g |
| Prescription 12 | 6.747 | 5.769 | 9.64 mg/g | 9.60 mg/g | 9.50 mg/g | 9.72 mg/g | 9.58 mg/g |
| Prescription 12 | 6.711 | | 9.65 mg/g | 9.63 mg/g | 9.60 mg/g | 9.61 mg/g | 9.59 mg/g |

The results show that the ruxolitinib composition prescription of the present invention did not experience an obvious change in the content of the ruxolitinib at different pH values ranging from 3 to 7, and had good stability.

**Example 4: Ruxolitinib gel and stability thereof**

Prescription 9, Prescription 10, and Prescription 12 in Example 2 were selected to prepare Gel prescription 1, Gel prescription 2, and Gel prescription 3, respectively. The total weight of the gels was 100 g.

The preparation method is as follows: mixing the ruxolitinib composition except the aqueous phase and heating to 45°C, stirring to make the excipients completely melted and the therapeutic agent dissolved, then uniformly stirring with swollen carbomer, followed by adding the aqueous phase to obtain the gel of the ruxolitinib composition.

The stability of the gel prescriptions provided in the present invention was investigated based on the content of ruxolitinib in Gel prescriptions 1, 2, and 3. Detailed results are shown in Table 6.

Table 6: Stability of Ruxolitinib Gel Prescription

| Batch NO. | pH | Day 0 | 1 M at 25°C | 2 M at 25°C | 1 M at 40°C | 2M at 40°C |
|---|---|---|---|---|---|---|
| | | Content (mg/g) | | | | |
| Gel prescription 1 | 6.239 | 9.99 | 10.14 | 10.12 | 9.95 | 10.01 |

(continued)

| Batch NO. | pH | Day 0 | 1 M at 25°C | 2 M at 25°C | 1 M at 40°C | 2M at 40°C |
|---|---|---|---|---|---|---|
| | | Content (mg/g) | | | | |
| Gel prescription 2 | 6.219 | 10.09 | 10.09 | 10.02 | 10.09 | 10.22 |
| Gel prescription 3 | 6.433 | 10.20 | 10.13 | 10.23 | 10.02 | 10.28 |

The results show that the pH values of Gel prescriptions 1, 2, and 3 were between 5 and 7, and the contents did not change significantly at both 25°C and 40°C, showing good temperature stability.

**Example 5: Study on *in Vitro* penetration and release of Ruxolitinib Gels**

Test method: A Franz diffusion cell (Tianjin Boyuweiye Technology Co., Ltd., Model TP-6) was used. An artificial membrane (Strat-M® Membrane) was fixed between the diffusion cell and a receiving cell, where the diffusion cell had a diameter of 1.5 cm, and the receiving cell had a volume of 15 mL. The diffusion cell was filled with about 300 mg of a ruxolitinib gel sample, and a receiving liquid (0.05 M phosphate buffer with a pH value of 7.2-7.4) was injected into the receiving cell. The water bath was at a temperature of 32 ± 1°C, and the magnetic stirring speed at constant temperature was 300 rpm/min. At 1 h, 2 h, 4 h, 6 h and 8 h, 1.5 mL of the receiving liquid was taken out from the receiving cell, and the same amount of the receiving liquid at the same temperature was supplemented into the receiving cell in time. The taken receiving liquid samples were centrifuged at a high speed (18000 rpm/min for 10 min), and the supernatant was taken out for determining the concentration ($C_n$) of the solution at different time points by HPLC.

The cumulative amount of the penetrated drug per unit area is calculated using the equation as follows:

$$Qn = (C_n V + \sum_{i=1}^{n} C_{i-1} V)/S$$

wherein

Qn: the cumulative amount of the penetrated drug per unit area at time point n (unit: mg/cm$^2$);
$C_n$: the drug concentration at time point n (unit: mg/mL);
$C_{i-1}$: the drug concentration at time point i-1 (unit: mg/mL);
V: the volume of the receiving cell (unit: mL); and
S: the effective penetration area which was 1.76625 cm$^2$ in this experiment.

A comparative example was prepared with reference to the prescription (wherein the concentration of the free base of ruxolitinib was 1.0%) shown in Table 4 of Example 3 of the specification of Chinese invention patent CN103002875 B granted to Incyte Holding Corporation.

Table 7: *In Vitro* Cumulative Released Amount of Prescriptions of the Invention (unit: mg/cm$^2$)

| | Gel prescription 1 | Gel prescription 2 | Gel prescription 3 | Comparative cream prescription |
|---|---|---|---|---|
| 30 min | 0.0010 | 0.0005 | 0.0081 | 0.0000 |
| 1 h | 0.0044 | 0.0016 | 0.0256 | 0.0003 |
| 2 h | 0.0181 | 0.0104 | 0.0652 | 0.0068 |
| 4 h | 0.0662 | 0.0354 | 0.1430 | 0.0294 |
| 6 h | 0.1119 | 0.0689 | 0.2101 | 0.0508 |
| 8 h | 0.1521 | 0.1086 | 0.2767 | 0.0694 |

From Table 8, it can be seen that compared to the comparative cream, Gel prescriptions 1, 2 and 3 exhibited faster release and significantly higher *in vitro* released amounts than the comparative cream.

**Example 6: Study on Efficacy of Ruxolitinib Gels on Psoriasis**

The drug of the present invention can effectively improve symptoms of psoriasis. The therapeutic effect of the drug of the present invention on psoriasis can be further confirmed by the following study.

Animal test: establishment of psoriasis vulgaris (PSO) mouse model and drug treatment

1. Drugs and reagents

Test materials: 48 healthy SPF level C57BL/6J mice (half male and half female, aged 8-9 weeks, and weighted 20-25 g); drug for modeling (5% imiquimod cream, Sichuan Mingxin Pharmaceuticals); positive control drugs (a commercially available calcipotriol ointment, and a commercially available Benvitimod cream); a blank gel preparation; a commercially available ruxolitinib phosphate cream (referred to as an original patented cream, ruxolitinib free base 1.5% ); Gel 1; and Gel 2, where the prescriptions of Gel 1 and Gel 2 are shown in Table 8.

Table 8: Prescriptions of Gel 1 and Gel 2 (Based on a Total Weight of 100 g)

| Function | Component | Gel 1 | Gel 2 |
|---|---|---|---|
| Active ingredient | Ruxolitinib (based on the free base) | 1.5% | 1.14% |
| Emulsifier | Polyoxyethylene hydrogenated castor oil (RH40) | 1% | 1% |
| | Polyoxyethylene hydrogenated castor oil (EL35) | 4% | 4% |
| Co-emulsifier | Propylene glycol monocaprylate (Capryol-90) | 3% | 3% |
| Solvent | Diethylene glycol monoethyl ether (HP) | 2% | 2% |
| | Propylene glycol | 30% | 30% |
| Gel matrix | Carbomer 980 | 0.5% | 0.5% |
| Chelating agent | Disodium edetate | 0.05% | 0.05% |
| pH regulator | Triethanolamine | 0.14% | 0.14% |
| Preservative | Methyl paraben | 0.12% | 0.12% |
| | Propyl paraben | 0.05% | 0.05% |
| Aqueous phase matrix | Purified water | added to 100% | added to 100% |

2. Test method

2.1 Grouping

Grouping: 8 test groups, 6 mice in each group and 48 mice in total.

1) Blank control group (no treatment except shaving) (N=6).
2) PSO model group (no treatment except smearing imiquimod for modeling) (N=6).
3) PSO model + blank gel preparation group (externally smeared twice a day, 15 mg/cm$^2$, treated for 7 consecutive days) (N=6).
4) PSO model + positive drug A group (treated with 0.005% calcipotriol ointment by externally smearing twice a day, 15 mg/cm$^2$, for 7 consecutive days) (N=6).
5) PSO model + positive drug B group (treated with 1% Benvitimod cream by externally smearing twice a day, 15 mg/cm$^2$, tentatively for 7 consecutive days) (N=6).
6) PSO model + Gel 1 sample (externally smeared twice a day, 15 mg/cm$^2$, treated for 7 consecutive days) (N=6).
7) PSO model + commercially available ruxolitinib cream (externally smeared twice a day, 15 mg/ cm$^2$, treated for 7 consecutive days) (N=6).
8) PSO model + Gel 2 sample (externally smeared twice a day, 15 mg/cm$^2$, treated for 7 consecutive days) (N=6).

Test steps:

Modeling: The mice were adaptively fed for 7 days to prepare a psoriasis vulgaris (PSO) mouse model.

The modeling method is as follows: A skin area (about 2 cm * 3 cm) on the back of the mice is prepared, 50 mg of 5% imiquimod cream was topically applied to the exposed skin every morning for 3 consecutive days for model preparation, and the application of the drug for modeling continued later.

Model validation: This model has mature SOP standard, and no more mice are set up for model validation.

Treatment: (3 days after modeling), treatment with a corresponding drug was performed on Day 4. During drug treatment, the administration of 5% IMQ continued to maintain modeling, and the administration of the drug for modeling continued according to the days of administration of the corresponding drug.

Group 1): The blank control group (no treatment) was not subjected to any treatment.

Group 2): The model group was smeared with 5% imiquimod cream at an amount of 62.5 mg per mouse for maintaining modeling, and not subjected to any therapeutic treatment.

Group 3): The model group was smeared with 5% imiquimod cream at an amount of 62.5 mg per mouse for maintaining modeling, additionally smeared with the blank gel 2 times every day, and subjected to the treatment for 7 consecutive days.

Group 4): The model group was smeared with 5% imiquimod cream at an amount of 62.5 mg per mouse for maintaining modeling, additionally smeared with the positive drug A (the calcipotriol ointment) 2 times every day, and subjected to the treatment for 7 consecutive days.

Group 5): The model group was smeared with 5% imiquimod cream at an amount of 62.5 mg per mouse for maintaining modeling, additionally smeared with the positive drug B (the Benvitimod cream) 2 times every day, and subjected to the treatment for 7 consecutive days.

Group 6): The model group was smeared with 5% imiquimod cream at an amount of 62.5 mg per mouse for maintaining modeling, additionally smeared with the tested drug (Gel 1 of this Example) 2 times every day, and subjected to the treatment for 7 consecutive days.

Group 7): The model group was smeared with 5% imiquimod cream at an amount of 62.5 mg per mouse for maintaining modeling, additionally smeared with the tested drug (the commercially available ruxolitinib phosphate cream) 2 times every day, and subjected to the treatment for 7 consecutive days. The sequence of administration was as follows: the therapeutic drug was administrated once in the morning, the drug for modeling was administrated once at noon, and the therapeutic drug was administrated once at night.

Group 8): The model group was smeared with 5% imiquimod cream at an amount of 62.5 mg per mouse for maintaining modeling, additionally smeared with the tested drug (Gel 2 of this Example) 2 times every day, and subjected to the treatment for 7 consecutive days.

Note:

In order to prevent drug licking between animals, the animals were separately placed in independent cages without padding for a period of time after the gel was smeared on the back to allow the gel to dry naturally (expected within 1 hour), and then put into the feeding cage for normal feeding. Individually feeding was prohibited since the animals were prone to depression; wrapping the applied area with gauze was prohibited since the drug could be absorbed.

2.2 Test results: pathological examination on PSO model animals treated with drugs

Test steps:

Psoriasis-like lesion area and severity index (PASI) scores of the mice in various groups were evaluated: changes in body weights and skin lesions of the mice in various groups were observed daily (weight determination: from adaptive feeding to the end of the administration, the weight were determined once a day per mouse with a total of 17 times; skin lesion scores: from the modeling period to the administration period, scores were evaluated daily once a day per mouse with a total of 10 times), and recorded daily by means of digital photography. According to the PASI scoring standard, the erythema, scales, and the degree of infiltration and thickening at skin lesions in the mice were scored 0-4 points; and the scores of the three were added to obtain a severity score of total skin lesions. After the scores of various groups of the mice were averaged, a trend line of the skin lesion scores was drawn to observe the changes in skin lesions in the mice in various groups.

2.3 Statistical method

Results were expressed as mean ± standard errors (Mean ± S.E.M.) and analyzed by statistical software SPSS 17.0. Differences between groups were compared using one-way ANOVA (homogeneity of variance not assumed, Dunnett's T3). $P<0.05$ indicates that the difference is statistically significant.

3. Results

Effects of the gels, the positive drugs and the commercially available ruxolitinib phosphate cream on skin lesion appearances (PASI) of imiquimod-induced psoriasis model mice.

PASI scores were evaluated according to the erythema, the scales and the degree of infiltration and thickening at skin lesions of the mice. From the 7-day score trend chart, it is found that after 4 days of administration, the PASI scores of the drug groups were decreased significantly, compared to the model group. On Day 4, the PASI scores of the Benvitimod group and the gel groups of the present invention were all decreased significantly as compared to the model group and also decreased significantly as compared to the blank gel group ($p < 0.05$, or $p < 0.01$). On Day 5 of the administration, the PASI scores of the gel groups of the present invention were decreased significantly as compared to the model group; and the PASI scores of the gel groups of the present invention were decreased significantly as compared to the blank gel. On Day 6 of the administration and Day 7 of the administration, the scores of the gel groups of the present invention were decreased significantly, compared to the model group and the blank gel group ($p < 0.01$). Details are shown in FIG. 1 and FIG. 2.

4. Conclusion

In this research, an imiquimod-induced psoriasis mouse model was used to evaluate the protective effects of the prescriptions of the present invention, the positive drugs and a commercially available comparative ruxolitinib cream. Results show that the gels of the present invention, the positive drugs and the commercially available ruxolitinib cream can all significantly reduce the PASI scores of the mice. Compared to the positive drugs and the commercially available ruxolitinib cream, the gels of the present invention has better effects, indicating that the gels of the present invention, the positive drugs and the commercially available ruxolitinib cream all have therapeutic effects for psoriasis, and that the effects of the gels of the present invention in topically treating psoriasis are better than the positive drugs and the commercially available ruxolitinib cream at the same concentration.

In the description of this specification, descriptions with reference to terms such as "one embodiment", "some embodiments", "example", "specific example" or "some examples" mean that the specific features, structures, materials or characteristics described in combination with the embodiments or the examples are included in at least one embodiment or example of the present invention. In the specification of the present invention, the illustrative representation of the above terms does not necessarily mean the same embodiment or example. Moreover, the specific features, structures, materials or characteristics may be combined in any suitable manner in one or more embodiments or examples. In addition, features of various embodiments or examples described in the specification may be connected or combined by those skilled in the art without contradicting each other.

While the embodiments of the present invention have been shown and described above, it should be understood that the above embodiments are illustrative, but cannot be construed as limiting the present invention. Changes, modifications, substitutions and variations may be made to the above embodiments by those of ordinary skill in the art within the scope of the present invention.

## Claims

1. A ruxolitinib composition, **characterized in that** the ruxolitinib composition contains a therapeutic agent and an emulsifier, wherein the therapeutic agent is ruxolitinib or a pharmaceutically acceptable salt thereof with a free base-based content of 0.5%-2.0% of the weight of the composition; the emulsifier is one or more of polyoxyethylene alkyl aryl ether, polyoxyethylene monolaurate, polyoxyethylene monolaurate, polyoxyethylene lauryl ether, polyoxyethylene hydrogenated castor oil, polyoxyethylene castor oil, polyoxyethylene plant oil, polyoxyethylene monooleate, polyoxyethylene monolaurate, and vitamin E succinate, preferably one or more of polyoxyethylene hydrogenated castor oil, polyoxyethylene castor oil, polyoxyethylene monooleate, polyoxyethylene monolaurate, and vitamin E succinate, and more preferably one or more of polyoxyethylene hydrogenated castor oil, polyoxyethylene castor oil, and vitamin E succinate.

2. The composition according to claim 1, **characterized in that** the emulsifier is one or more selected from polyoxyethylene hydrogenated castor oil, polyoxyethylene castor oil, and vitamin E succinate, wherein the polyoxyethylene hydrogenated castor oil is one or more selected from RH20, RH30, RH40, RH50, and RH60, preferably one of RH40 and RH60, and more preferably RH40; the polyoxyethylene castor oil is one or more selected from EL20, EL25, EL30, EL35, EL40, and EL60, preferably one or more of EL30, EL35, and EL40, and more preferably EL35; the vitamin E succinate is one or more selected from VE-TPGS 400, VE-TPGS 1000, and VE-TPGS1500, preferably VE-TPGS 1000; and the content of the emulsifier is 2%-30%, further preferably 3%-12% of the weight of the composition.

**3.** The ruxolitinib composition according to claim 2, **characterized in that** the composition contains a co-emulsifier, which is propylene glycol monocaprylate, with a content of 0-10%, preferably 0-7% of the weight of the composition.

**4.** The ruxolitinib composition according to claim 3, **characterized in that** the composition contains a solvent, which is one or more selected from propylene glycol, diethylene glycol monoethyl ether, and ethanol, with a content of 0-60%, preferably 0-50% of the weight of the composition.

**5.** The ruxolitinib composition according to claim 4, **characterized in that** the composition contains an aqueous phase, which contains water and optionally excipients, wherein the excipients are selected from one or more of a pH regulator, an osmotic pressure regulator, a preservative, and an opacifying agent; and the sum of the contents of the aqueous phase, the therapeutic agent, the emulsifier, the co-emulsifier and the solvent is 100% by the weight of the composition.

**6.** A method for the preparation of the ruxolitinib composition of any one of claims 1-5, **characterized in that** the method comprises the steps of:
heating the therapeutic agent, the emulsifier, the co-emulsifier and/or the solvent to 30-70°C, preferably 35-60°C, stirring, followed by adding the aqueous phase to obtain the ruxolitinib composition.

**7.** A method for the preparation of the ruxolitinib composition of any one of claims 1-5, **characterized in that** the method comprises the steps of:
mixing the therapeutic agent, the emulsifier, the co-emulsifier and/or the solvent, heating them to 30-70°C, preferably 35-60°C while stirring until dissolved, then adding the emulsifier, stirring, followed by adding the aqueous phase to obtain the ruxolitinib composition.

**8.** Use of the ruxolitinib composition of any one of claims 1-5 in the preparation of a pharmaceutical formulation, wherein the pharmaceutical formulation is selected from a capsule, a tablet, granules, micro pills, an ointment, a cream, and a gel.

**9.** A ruxolitinib gel, **characterized in that** the gel contains the composition of any one of claims 1-5, and a gel material.

**10.** The ruxolitinib gel according to claim 9, wherein the gel material is one or more selected from carbomer, gellan gum, guar gum, sodium carboxymethyl cellulose and hydroxypropyl cellulose, preferably carbomer, with a content of 0.10%-2.0%, further preferably 0.25%-1.0% of the weight of the gel.

**11.** A ruxolitinib gel, **characterized in that** the ruxolitinib gel contains:

a therapeutic agent: ruxolitinib, or a pharmaceutically acceptable salt thereof;
an emulsifier: one or more of polyoxyethylene hydrogenated castor oil, polyoxyethylene castor oil and vitamin E succinate;
a co-emulsifier: propylene glycol monocaprylate;
a solvent: one or more of propylene glycol, diethylene glycol monoethyl ether, and ethanol;
a gel material: one or more of carbomer, methylcellulose, sodium carboxymethyl cellulose, chitosan, gellan gum, guar gum, sodium carboxymethyl cellulose, and hydroxypropyl cellulose; and
water q.s. to 100wt%.

**12.** The ruxolitinib gel according to claim 11, **characterized in that** the ruxolitinib gel contains:

a therapeutic agent: 0.5%-2.0% of ruxolitinib, or a pharmaceutically acceptable salt thereof (based on the free base);
an emulsifier: 2%-30% of one or more of polyoxyethylene hydrogenated castor oil, polyoxyethylene castor oil, and vitamin E succinate;
a co-emulsifier: 0-20% of propylene glycol monocaprylate;
a solvent: 0-60%, preferably 0-50%, more preferably 2%-50% of one or more of propylene glycol, diethylene glycol monoethyl ether, and ethanol;
a gel material: 0.10%-2.0% of one or more of carbomer, methylcellulose, sodium carboxymethyl cellulose, chitosan, gellan gum, guar gum, sodium carboxymethyl cellulose, and hydroxypropyl cellulose; and
water q.s. to 100wt%;
based on the total weight of the gel;

peferably, the ruxolitinib gel contains:

a therapeutic agent: 0.5%-2.0% of ruxolitinib, or a pharmaceutically acceptable salt thereof (based on the free base);
an emulsifier: 1%-10% of polyoxyethylene hydrogenated castor oil, 1%-20% of polyoxyethylene castor oil, and 0-20% of vitamin E succinate;
a co-emulsifier: 0-20% of propylene glycol monocaprylate;
a solvent: 1%-50% of propylene glycol, 1%-10% of diethylene glycol monoethyl ether, and 0-50% of ethanol;
a gel material: 0.10%-2.0% of carbomer; and
water q.s. to 100wt%;
based on the total weight of the gel;
more preferably, the ruxolitinib gel contains:

a therapeutic agent: 0.5%-2.0% of ruxolitinib, or a pharmaceutically acceptable salt thereof (based on the free base);
an emulsifier: 1.0%-5.0% of polyoxyethylene hydrogenated castor oil, 1%-10% of polyoxyethylene castor oil, and 0-10% of vitamin E succinate;
a co-emulsifier: 0-10% of propylene glycol monocaprylate;
a solvent: 1%-45% of propylene glycol, 1%-5.0% of diethylene glycol monoethyl ether, and 0-40% of ethanol;
a gel material: 0.10%-2.0% of carbomer; and
water q.s. to 100wt%;
based on the total weight of the gel;
further preferably, the ruxolitinib gel contains:

a therapeutic agent: 0.5%-2.0% of ruxolitinib, or a pharmaceutically acceptable salt thereof (based on the free base);
an emulsifier: 1%-2% of polyoxyethylene hydrogenated castor oil, 4%-10% of polyoxyethylene castor oil, and 0-5% of vitamin E succinate;
a co-emulsifier: 0-7% of propylene glycol monocaprylate;
a solvent: 5%-45% of propylene glycol, 2%-5% of diethylene glycol monoethyl ether, and 0-15% of ethanol;
a gel material: 0.25%-1.0% of carbomer; and
water q.s. to 100wt%;
based on the total weight of the gel.

**13.** A method for the preparation of the ruxolitinib gel of any one of claims 9-12, wherein the method comprises the steps of:
mixing the ruxolitinib composition except the aqueous phase and heating to 30-70°C, preferably 35-60°C, stirring to make the excipient completely melted and the therapeutic agent dissolved, then uniformly stirring with swollen carbomer, followed by adding the aqueous phase to obtain the ruxolitinib gel.

**14.** Use of the ruxolitinib composition of any one of claims 1-5 for treating a disease, wherein the disease is one or more of dermatitis, psoriasis, vitiligo, hydradenitis, urticaria, and alopecia areata.

**15.** Use of the ruxolitinib gel of any one of claims 9-12 for treating a disease, wherein the disease is one or more of dermatitis, psoriasis, vitiligo, hydradenitis, urticaria, and alopecia areata.

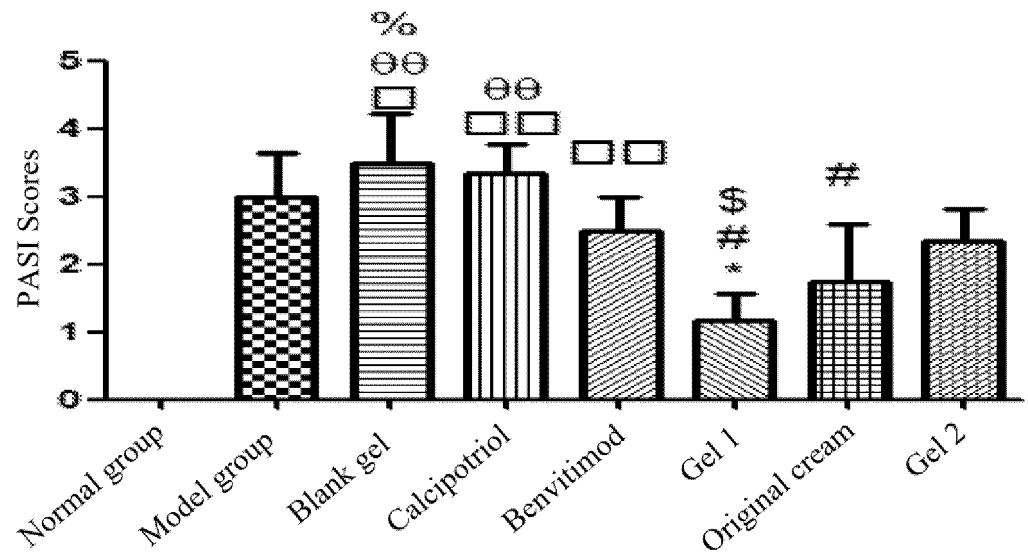
PASI Scores
0
1
2
3
4
5
Normal group
Model group
Blank gel
Calcipotriol
Benvitimod
Gel 1
Original cream
Gel 2
%
θθ
θθ
* # $
#

FIG.1

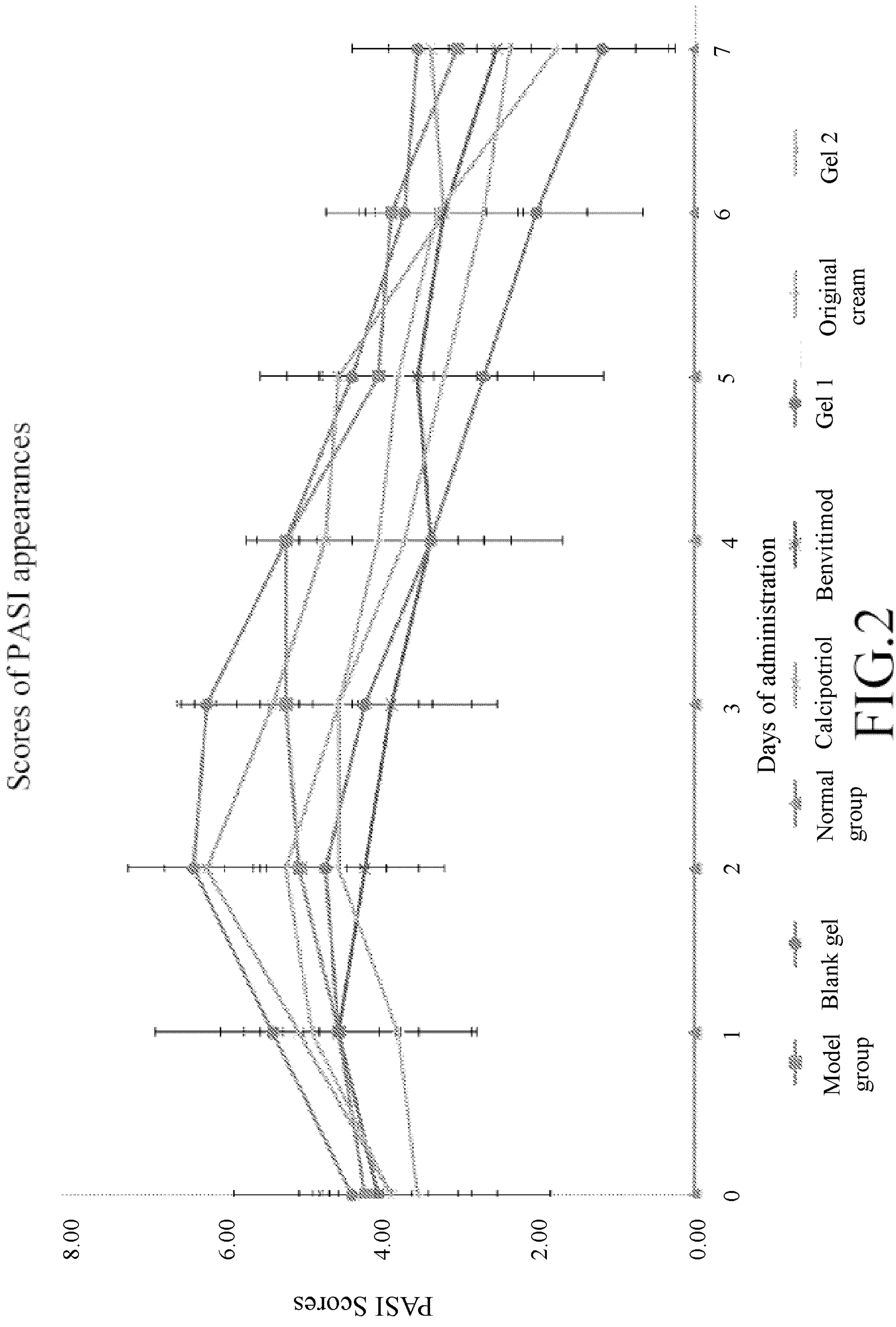
Scores of PASI appearances
PASI Scores
8.00
6.00
4.00
2.00
0.00
0
1
2
3
4
5
6
7
Days of administration
Model group
Blank gel
Normal group
Calcipotriol
Benvitimod
Gel 1
Original cream
Gel 2

FIG.2

## INTERNATIONAL SEARCH REPORT

International application No.
**PCT/CN2022/126399**

**A. CLASSIFICATION OF SUBJECT MATTER**

A61K 31/519(2006.01)i; A61K 47/44(2017.01)i; A61K 47/24(2006.01)i; A61K 47/10(2006.01)i; A61K 9/06(2006.01)i; A61P 19/08(2006.01)i; A61P 7/00(2006.01)i

According to International Patent Classification (IPC) or to both national classification and IPC

**B. FIELDS SEARCHED**

Minimum documentation searched (classification system followed by classification symbols)

A61K A61P

Documentation searched other than minimum documentation to the extent that such documents are included in the fields searched

Electronic data base consulted during the international search (name of data base and, where practicable, search terms used)

CNTXT; ENTXTC; VCN; CJFD; DWPI; CNKI; WFNPL; NPTXT; NPABS; Web of Science; STNext; 读秀, DUXIU: 鲁索利替尼, 鲁索替尼, 卢佐替尼, 芦可替尼, 卢克替尼, 乳膏, 乳剂, 乳液, 乳化剂, 聚氧乙烯, 聚环氧乙烷, 维生素E琥珀酸, 凝胶, ruxolitinib, Jakafi, INC 424, INCB 018424, INC424, RUXO, Jakavi, PEO, VE-TPGS, gel, Cream, emulsion, lotion, emulsifier

**C. DOCUMENTS CONSIDERED TO BE RELEVANT**

| Category* | Citation of document, with indication, where appropriate, of the relevant passages | Relevant to claim No. |
|---|---|---|
| A | US 2020069965 A1 (YALE UNIVERSITY et al.) 05 March 2020 (2020-03-05) description, paragraphs [0006]-[0008], [0087], and [0107] | 1-15 |
| PX | US 2022202834 A1 (INCYTE CORP.) 30 June 2022 (2022-06-30) description, paragraphs [0051] and [1117]-[1124] | 1-8, 14 |

☐ Further documents are listed in the continuation of Box C. ☑ See patent family annex.

* Special categories of cited documents:
"A" document defining the general state of the art which is not considered to be of particular relevance
"E" earlier application or patent but published on or after the international filing date
"L" document which may throw doubts on priority claim(s) or which is cited to establish the publication date of another citation or other special reason (as specified)
"O" document referring to an oral disclosure, use, exhibition or other means
"P" document published prior to the international filing date but later than the priority date claimed
"T" later document published after the international filing date or priority date and not in conflict with the application but cited to understand the principle or theory underlying the invention
"X" document of particular relevance; the claimed invention cannot be considered novel or cannot be considered to involve an inventive step when the document is taken alone
"Y" document of particular relevance; the claimed invention cannot be considered to involve an inventive step when the document is combined with one or more other such documents, such combination being obvious to a person skilled in the art
"&" document member of the same patent family

| Date of the actual completion of the international search | Date of mailing of the international search report |
|---|---|
| **07 December 2022** | **14 December 2022** |

| Name and mailing address of the ISA/CN | Authorized officer |
|---|---|
| **China National Intellectual Property Administration (ISA/CN)**<br>**No. 6, Xitucheng Road, Jimenqiao, Haidian District, Beijing 100088, China** | |
| Facsimile No. **(86-10)62019451** | Telephone No. |

Form PCT/ISA/210 (second sheet) (January 2015)

**INTERNATIONAL SEARCH REPORT**

International application No.
**PCT/CN2022/126399**

**Box No. II Observations where certain claims were found unsearchable (Continuation of item 2 of first sheet)**

This international search report has not been established in respect of certain claims under Article 17(2)(a) for the following reasons:

1. ☑ Claims Nos.: **14、15**
because they relate to subject matter not required to be searched by this Authority, namely:

[1] The use involved in claims 14 and 15 relates to a disease treatment method implemented on a living human or animal body and directly aiming at treating diseases, which belongs to a method for treating a human or animal body, and belongs to the cases listed in PCT Rule 39.1(iv) for which no international search is required. The search and opinions for claims 14 and 15 were formed on the basis of the following amendments:

[2] **claim**14: a use of the ruxolitinib composition according to one of claims 1-5 in the preparation of a drug for treating diseases, the diseases being one or more of dermatitis, psoriasis, vitiligo, hidradenitis, urticaria, and alopecia areata; and

[3] **claim**15: a use of the ruxolitinib gel according to claims 9-12 in the preparation of a drug for treating diseases, the diseases being one or more of dermatitis, psoriasis, vitiligo, hidradenitis, urticaria, and alopecia areata.

2. ☐ Claims Nos.:
because they relate to parts of the international application that do not comply with the prescribed requirements to such an extent that no meaningful international search can be carried out, specifically:

3. ☐ Claims Nos.:
because they are dependent claims and are not drafted in accordance with the second and third sentences of Rule 6.4(a).

Form PCT/ISA/210 (continuation of first sheet) (January 2015)

**INTERNATIONAL SEARCH REPORT**
**Information on patent family members**

International application No.
**PCT/CN2022/126399**

| Patent document cited in search report | Publication date (day/month/year) | Patent family member(s) | Publication date (day/month/year) |
|---|---|---|---|
| US 2020069965 A1 | 05 March 2020 | WO 2018200786 A1 | 01 November 2018 |
| US 2022202834 A1 | 30 June 2022 | WO 2022120131 A1 | 09 June 2022 |

Form PCT/ISA/210 (patent family annex) (January 2015)

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description


- CN 103002875 B
- US 20200405627 A1
- CN 111337640 A